# EUROPÄISCHE PATENTSCHRIFT

(11) **EP 1 760 065 B1**
(45) Veröffentlichungstag und Bekanntmachung des Hinweises auf die Patenterteilung: **18.03.2009**
(21) Anmeldenummer: 06015532.2
(22) Anmeldetag: 26.07.2006
(51) Int. Cl.: C07C 67/055, C07C 69/15

(54) **Verfahren zur Isolierung von Vinylacetat**
Process for isolating vinyl acetate
Procédé d'isolement de l'acétate de vinyle

(30) Priorität: 05.08.2005 DE 102005036930
(43) Veröffentlichungstag der Anmeldung: 07.03.2007
(73) Patentinhaber: Celanese Chemicals Europe GmbH, 61476 Kronberg (DE)
(72) Erfinder: Stamm, Johann, 65929 Frankfurt (DE); Rinne, Bernd, 65929 Frankfurt (DE); Hess, Stefan, Dr., 64521 Gross-Gerau (DE); Sachs, Hans-Jochen, 65933 Frankfurt (DE); Sehr, Michael, 65551 Limburg (DE); Bayer, Michael J., Dr., 65760 Eschborn (DE); Nuber, Berthold, Dr., 65933 Frankfurt (DE); Wagner, Martin, 65929 Frankfurt (DE)
(74) Vertreter: Weber, Thomas

(56) Entgegenhaltungen:
- EP-A2- 0 423 658

## Beschreibung

Die vorliegende Erfindung betrifft ein Verfahren zur Isolierung von Vinylacetat aus dem Gasgemisch, das bei der Umsetzung von Ethylen mit Essigsäure und Sauerstoff aus Palladium oder Palladiumverbindungen enthaltenden Katalysatoren in der Gasphase gebildet wird, unter Rückführung der im Kreisgaswäscher anfallenden essigsauren Lösung in die erste Destillationskolonne.

Die Herstellung von Vinylacetat durch Umsetzung von Ethylen mit Essigsäure und Sauerstoff oder sauerstoffhaltigen Gasen an Festbettkatalysatoren in der Gasphase ist bereits bekannt. Die Reaktion erfolgt im allgemeinen bei Drücken von 1 bis 2,5 MPa und Temperaturen von 100 bis 250°C. Geeignete Katalysatoren enthalten einen Edelmetallanteil und einen Aktivatoranteil. Der Edelmetallanteil besteht aus Palladium und/oder dessen Verbindungen; zusätzlich können noch Gold oder dessen Verbindungen anwesend sein. Der Aktivatoranteil besteht aus Verbindungen von Elementen der 1. Hauptgruppe und/oder der 2. Hauptgruppe und/oder Cadmium. Diese aktiven Komponenten werden auf Träger in feiner Verteilung aufgebracht, wobei als Tägermaterial im allgemeinen Kieselsäure oder Aluminiumoxid verwendet wird.

Im allgemeinen liegt der Palladiumgehalt im Katalysator zwischen 0,5 und 5 Gew.-%.

Wird Gold bzw. eine seiner Verbindungen eingesetzt, so wird es in einem Anteil von 0,01 bis 4 Gew.-% zugegeben.

Jeder einzelne Aktivator wird im allgemeinen ebenfalls in einem Anteil von 0,01 bis 4 Gew.-% zugegeben. Bei allen drei Prozentangaben wird jeweils der Metallanteil der Komponente auf die Gesamtmasse des Trägerkatalysators bezogen. Bevorzugt sind folgende Katalysatoren: Palladium/Alkalielement/Cadmium sowie Palladium/Gold/Alkalielement, wobei Palladium bzw. Gold als Metalle oder Verbindungen im fertigen Katalysator vorliegen können und wobei als Alkalielement Kalium bevorzugt ist. Kalium wird in Form eines Carboxylats, insbesondere als Acetat, eingesetzt.

Besonders bevorzugt sind die Katalysatoren Palladiumacetat/Kaliumacetat/Cadmiumacetat, sowie Palladiumacetat/Bariumacetoaurat/Kaliumacetat.

In dem mehrstufigen katalytischen Prozess entstehen Vinylacetat und Wasser in äquimolaren Mengen, wie in der folgenden Bruttogleichung dargestellt:

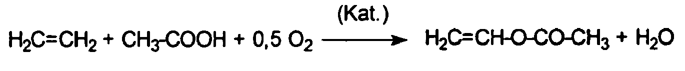

Aus der nicht ganz zu vermeidenden Totaloxidation des Ethylens entsteht CO₂ und Wasser:

H₂C=CH₂ + 3 O₂ → 2 CO₂ + 2 H₂O

Es fällt daher mehr als 1 mol Wasser pro mol Vinylacetat an; im allgemeinen macht das Gewicht des Wassers ca. ein Vierteil des Gewichts des gebildeten Vinylacetats aus.

Außer CO₂ bilden sich in geringer Menge noch andere Nebenprodukte, darunter auch Ethylacetat, in einem Anteil von etwa 1000-2000 Gew.-ppm, bezogen auf das gebildete Vinylacetat.

Ethylacetat darf in reinem Vinylacetat nur in einem geringen Anteil von höchstens 250 Gew.-ppm enthalten sein. Die Abtrennung von Ethylacetat erfordert einen hohen Energieaufwand und der Stand der Technik behandelt verschiedene Verfahren, den Energieaufwand bei der Reinigung von Vinylacetat unter Abtrennung von Ethylacetat und anderen Nebenprodukten zu reduzieren.

Das zur Reaktion eingesetzte Gemisch enthält ein mehrfaches der stöchiometrisch erforderlichen Menge an Ethylen. Dementsprechend ist der Ethylenumsatz mit etwa 10% relativ gering und das nicht umgesetzte Ethylen muss in die Reaktionszone zurückgeführt werden. Vinylacetat wird üblicherweise in einem mehrstufigen Verfahren aus dem Gemisch der gasförmig anfallenden Reaktionsprodukte abgetrennt.

Bei dem Verfahren gemäß DE-A1-3 422 575 wird das den Vinylacetatreaktor verlassende heiße Gasgemisch, das im wesentlichen aus Ethylen, Essigsäure, Vinylacetat, Wasser, Kohlendioxid, Sauerstoff und Inerten, wie beispielsweise Stickstoff und Argon, besteht und das Ethylacetat enthält, in eine ohne zusätzliche Beheizung arbeitende erste Destillationskolonne eingeleitet, in die so genannte Vorentwässerungskolonne. Das am Kopf dieser Kolonne austretende Gasgemisch wird zunächst in einem Wärmetauscher mit dem Rücklauf auf die Vorentwässerungskolonne in Kontakt gebracht, wobei sich das Gasgemisch abkühlt und der Rücklauf entsprechend erwärmt. Das Gasgemisch gelangt anschließend aus dem Wärmetauscher in einen Kondensator. Die dabei verflüssigten Anteile werden in einem Sammelbehälter aufgefangen, in dem es zur Trennung in die wässrige und organische Phase kommt. Die wässrige Phase wird ausgeschleust während die organische Phase ganz oder teilweise als Rücklauf auf den Kopf der Vorentwässerungskolonne zurückgeführt wird.

Die nicht im Kondensator verflüssigten Anteile enthalten noch gasförmiges Vinylacetat. Dieses wird in einer mit Essigsäure als Waschflüssigkeit betriebenen Waschkolonne, die man auch als Kreisgaswäscher bezeichnet, aus dem Gasgemisch herausgewaschen. Das verbleibende Restgas wird zum Reaktor zurückgeführt. Der Sumpfablauf des Kreisgaswäschers sowie der Rest der aus dem Kondensat der Vorentwässerungskolonne verflüssigten organischen Phase wird in einem weiteren Behälter gesammelt, sofern nicht die gesamte aus dem Kondensat verflüssigte organische Phase als Rücklauf in der Vorentwässerungskolonne verwendet wird.

Am Sumpf der Vorentwässerungskolonne fällt ein Gemisch an, das aus Vinylacetat, Essigsäure und etwa der Hälfte des Reaktionswassers sowie Nebenprodukten besteht. Die andere Hälfte des Reaktionswassers ist bereits ohne Energiezufuhr abgetrennt worden und bildet die wässrige Phase des Kondensats, das bei der Abkühlung des Kopfdampfes der Vorentwässerungskolonne entsteht.

Das Sumpfprodukt der Vorentwässerungskolonne wird zunächst einem Sammelbehälter zugeführt, den man auch als Rohvinylacetatsammelbehälter bezeichnet, und anschließend in einer zweiten Destillationskolonne, der sogenannten Azeotropkolonne aufgearbeitet. Es fällt wassergesättigtes Vinylacetat als Kopfprodukt, ein Ethylacetat haltiger Seitenstrom sowie ein Sumpfprodukt an, das als Rückessigsäure in das System zurückgeführt wird. Der Ethylacetat haltige Seitenstrom wird ausgeschleust. Das wassergesättigte Vinylacetat, das nicht als Rücklauf auf den Kopf der zweiten Destillationskolonne gegeben wird, wird mit dem Sumpfablauf des Kreisgaswäschers und dem Rest der aus dem Kondensat der Vorentwässerungskolonne verflüssigten organischen Phase zusammengeführt.

Anschließend wird das Gemisch auf eine weitere dritte Destillationskolonne, auf die sogenannte Entwässerungskolonne gegeben. Der Kopfdampf an dieser Kolonne wird nach Kondensation nahezu vollständig als Rücklauf zurückgeführt. Der Seitenstromabzug wird in eine wässrige und organische Phase getrennt, wobei dann die wässrige Phase ausgeschleust und die organische Phase wieder auf die Kolonne gegeben wird. Am Boden der Entwässerungskolonne wird ein trockenes Vinylacetat/Essigsäuregemisch abgezogen und auf eine weitere vierte Kolonne, auf die sogenannte Reinvinylacetatkolonne gegeben. Bei dieser Kolonne fällt als Kopfprodukt nahezu ethylacetatfreies Vinylacetat an, während der Sumpf dieser Kolonne, der Essigsäure, Hochsieder und Spuren von Vinylacetat und Ethylacetat enthält, unter Ausschleusen eines Teilstromes wieder in den Prozess zurückgeführt wird.

Eine weitere Variante des bekannten Verfahrens zur Aufarbeitung von Vinylacetat ist aus EP-A2-0 423 658 bekannt. Danach wird das Sumpfprodukt des Kreisgaswäschers nicht unmittelbar mit dem an der Azeotropkolonne anfallenden wasserhaltigen Vinylacetat zusammengeführt sondern zuerst in eine weitere Kolonne eingeleitet, in der als Kopfprodukt ein Vinylacetat-Wasser-Azeotrop und als Sumpfprodukt Essigsäure anfällt, die in den Prozess zurückgeführt wird. Das in dieser zusätzlichen Kolonne anfallende wässrige Vinylacetat wird mit dem aus der Azeotropkolonne erhaltenen wassergesättigtem Vinylacetat vereinigt und entsprechend dem Verfahren nach DE-A1-3 422 575 in der nachgeschalteten Entwässerungskolonne und Reinvinylacetatkolonne aufgearbeitet. Das Verfahren gemäß EP-A2-0 423 658 erfordert zur Ethylacetatabtrennung etwa denselben Aufwand an Destillationsenergie wie das Verfahren gemäß DE-A1-3 422 575, allerdings ist eine geringere Zahl an Kolonnenböden erforderlich, was weniger Investitionskosten bedeutet. Der nicht kondensierte Teil des Vinylacetats aus der Vorentwässerungskolonne, der in dem Kreisgaswäscher mit Essigsäure herausgewaschen wird und als essigsaure Lösung anfällt, sowie die organische Phase des Kondensats aus der Vorentwässerungskolonne enthalten nahezu kein Ethylacetat mehr und eine energetisch aufwendige Befreiung dieser Vinylacetatströme von Ethylacetat entfällt. Allerdings erfordert diese Verfahrensvariante den Betrieb einer zusätzlichen Destillationskolonne zur Auftrennung des Sumpfablaufs aus dem Kreisgaswäscher.

Die bekannten Aufarbeitungsverfahren zur Gewinnung von reinem Vinylacetat weisen immer noch gewisse Nachteile auf. So enthält der Sumpfablauf des Kreisgaswäschers und der Sumpfablauf der Vorentwässerungskolonne erhebliche Mengen an Gasen gelöst, vor allem an Ethylen. Bei der Entspannung des Sumpfablaufs aus der Vorentwässerungskolonne und aus dem Kreisgaswäscher in dem Rohvinylacetatsammelbehälter wird daher eine beachtliche Menge Rückgas frei, die unter hohem Energieaufwand in einem Rückgasverdichter aufzukomprimieren ist, bevor sie wieder in den Reaktionskreislauf zurückgeführt werden kann. Im Allgemeinen wird das rohe Vinylacetat von einem Druck im Bereich von 0,5 bis 2,0 MPa auf eine Druckstufe von 0,02 bis 0,2 MPa entspannt. Das bei der Entspannung gebildete Gas enthält überwiegend Ethylen sowie Kohlendioxid, Stickstoff und weitere Inerte wie Argon sowie organische Bestandteile wie Essigsäure und geringe Mengen an Vinylacetat und Ethylacetat. Man bezeichnet dieses Gas auch als Rückgas, das in den Prozess zurückgeführt wird.

Charakteristisch für den bekannten Aufarbeitungsprozess ist das Zusammenführen der aus dem Sumpfablauf des Kreisgaswäschers anfallenden essigsauren Lösung mit dem wassergesättigten Vinylacetat aus dem Kopfprodukt der Azetropkolonne und dem Rest der aus dem Kondensat der Vorentwässerungskolonne verflüssigten organischen Phase. Daher wird den weiteren Reinigungsstufen, die in der nachgeschalteten Entwässserungskolonne und Reinvinylacetatkolonne erfolgen, ein essigsaures Gemisch zugeführt, aus dem unter hohem Energieaufwand Essigsäure abgetrennt werden muss. Zudem müssen Entwässerungskolonne und Reinvinylacetatkolonne mit korrosionsbeständigen Materialien, die gegenüber Essigsäure unempfindlich sind, ausgelegt werden.

Ebenfalls enthält das Kondensat der Vorentwässerungskolonne, das nicht als Rücklauf auf den Kopf der Vorentwässerungskolonne zurückgegeben wird, noch eine gewisse Menge Ethylacetat. Da dieser Stoffstrom erst nach der Azeotropkolonne mit dem dort als Kopfprodukt anfallenden, wassergesättigten Vinylacetat zusammengeführt wird, wird der nachgeschalteten Entwässerungs- und Reinvinylacetatkolonne ein ethylacetathaltiger Stoffstrom zugeführt, aus dem Ethylacetat nur unter hohem Energieaufwand abgetrennt werden kann.

Schließlich ist eine verbesserte Abtrennung von Wasser und Ethylacetat an einer möglichst vorgeschalteten Aufarbeitungsstufe erstrebenswert, um das Durchschleppen dieser unerwünschten Stoffe durch den gesamten Aufarbeitungsprozess möglichst zu unterbinden und die damit verbundene, energetisch aufwendige Abtrennung bei der Reinvinylacetatdestillation zu vermeiden.

Die Erfindung besteht daher in einem Verfahren zur Abtrennung von Vinylacetat aus dem Gasgemisch, das bei der Umsetzung von Ethylen mit Essigsäure und Sauerstoff an Palladium oder Palladiumverbindungen enthaltenden Katalysatoren in der Gasphase gebildet wird, wobei man
a) das aus der Reaktionszone austretende Gasgemisch in eine erste Destillationskolonne einleitet,
b) das am Kopf der ersten Destillationskolonne austretende Gasgemisch auf -20 bis +50°C abkühlt, wobei sich das anfallende Kondensat in eine Wasserphase und in eine organische Phase trennt,
c) die in Schritt b) gebildete Wasserphase abzieht,
d) die in Schritt b) gebildete organische Phase ganz oder teilweise als Rücklauf auf den Kopf der in Schritt a) benutzten ersten Destillationskolonne zurückführt und einen nicht als Rücklauf verwendeten Teil der organischen Phase abzieht,
e) das in Schritt b) nicht kondensierte, Vinylacetat haltige Gas in einer Waschkolonne mit mindestens 90%iger wässriger Essigsäure wäscht und dabei am Sumpf eine Vinylacetat haltige, essigsaure Lösung gewinnt,
f) das Vinylacetat, Ethylacetat, Essigsäure und Wasser enthaltende Sumpfprodukt von Schritt a) einem Sammelbehälter zuführt und die unter Druck stehende Flüssigkeit entspannt, wobei sich ein Gas bildet,
g) die bei der Entspannung in Schritt f) anfallende Flüssigkeit einer zweiten Destillationskolonne zuführt und aus einer Anreicherungszone oberhalb von deren Sumpf einen Ethylacetat haltigen Seitenstrom abzieht,
h) das Essigsäure und Wasser enthaltende Sumpfprodukt von Schritt g) ganz oder teilweise zur Gaswäsche in Schritt e) benutzt,
i) den Kopfdampf von Schritt g) abkühlt, wobei sich das anfallende Kondensat in eine wässrige und eine organische Phase trennt,
j) die in Schritt i) gebildete wässrige Phase abzieht,
k) einen Teil der in Schritt i) gebildeten organischen Phase als Rücklauf auf den Kopf der in Schritt g) benutzten zweiten Destillationskolonne zurückführt und den restlichen Teil abzieht, dadurch gekennzeichnet, dass man
l) einen Teil des Sumpfproduktes der in Schritt e) benutzten Waschkolonne unter Umpump zunächst kühlt und in den Sumpf der in Schritt e) benutzten Waschkolonne zurückführt, den restlichen Teil abzieht und den abgezogenen Teil auf eine Temperatur von wenigstens 30°C erwärmt und auf den unteren Teil der in Schritt a) benutzten ersten Destillationskolonne gibt,
m) die in Schritt d) abgezogene restliche organische Phase entspannt, das bei der Entspannung gebildete Gas mit dem in Schritt f) gebildeten Gas vereinigt und das gemeinsame Gas wieder in den Prozess zurückführt,
n) die in Schritt m) anfallende organische Phase mit der in Schritt i) anfallenden organischen Phase vereinigt und den in Schritt k) restlichen abgezogenen, nicht als Rücklauf verwendeten Teil der organischen Phase in eine dritte Destillationskolonne einleitet,
o) das Kopfprodukt der dritten Destillationskolonne aus Schritt n) abkühlt und die dabei anfallenden Leichtsieder und das dabei anfallende Wasser abtrennt,
p) das Sumpfprodukt der dritten Destillationskolonne aus Schritt n) in eine vierte Destillationskolonne einleitet,
q) am Kopf der in Schritt p) verwendeten vierten Destillationskolonne reines Vinylacetat abzieht.

In Schritt a) wird vorzugsweise das aus der Reaktionszone austretende Gasgemisch zunächst im Gegenstromwärmeaustausch mit dem kälteren Kreisgas, das damit aufgeheizt und dann zur Reaktion zurückgeführt wird, auf 115°C-150°C abgekühlt. Dabei tritt noch keine Kondensation der verflüssigbaren Anteile ein und das Gasgemisch wird in die erste Destillationskolonne, auch Vorentwässerungskolonne genannt, eingeleitet.

Die Menge der in Schritt b) gebildeten organischen Phase ist abhängig davon, bis zu welcher Temperatur in diesem Schritt abgekühlt wird. Denjenigen Teil der organischen Phase aus Schritt b), den man für Schritt d) nicht als Rücklauf benutzt, zieht man ab und entspannt in Schritt m) von einer Druckstufe von 0,5 bis 2,0 MPa auf eine Druckstufe von 0,02 bis 0,2 MPa, vorzugsweise auf 0,1 bis 0,15 MPa. Die dabei anfallende Flüssigkeit vereinigt man in Schritt n) mit der organischen Phase aus dem kondensierten Kopfprodukt der zweiten Destillationskolonne, auch Azeotropkolonne genannt (Schritt i). Vorzugsweise werden beide organische Phasen im Phasentrenner der Azeotropkolonne vereinigt. Der in Schritt n) nicht als Rücklauf auf den Kopf der Azeotropkolonne gegebene Anteil der organischen Phase wird in eine dritte Destillationskolonne, die man auch als Entwässerungskolonne bezeichnet, eingeleitet.

Vorzugsweise wählt man die Abkühlungstemperatur in Schritt b) und den als Rücklauf in Schritt d) benutzten Anteil der in b) gebildeten organischen Phase derart, dass möglichst wenig Vinylacetat, aber möglichst das gesamte Ethylacetat im Sumpfprodukt von Schritt a) enthalten sind.

Charakteristisch für die erfindungsgemäße Arbeitsweise ist der Betrieb der in Schritt e) verwendeten Waschkolonne und die Rückführung des Sumpfablaufs aus der Waschkolonne auf den unteren Teil der in Schritt a) verwendeten ersten Destillationskolonne. Ein Teil des Sumpfabzugs aus der Waschkolonne, die man auch als Kreisgaswäscher bezeichnet, wird im Umpump geführt, wobei der im Umpump geführte Teil des Sumpfproduktes aus der Waschkolonne abgekühlt wird. Zur Abkühlung des Sumpfproduktes werden Mittel verwendet, die dem Fachmann gebräuchlich sind, beispielsweise Wärmetauscher. Der nicht in den Umpump geführte Teil des Sumpfproduktes wird aus der Waschkolonne abgezogen, auf eine Temperatur von wenigstens 30°C, vorzugsweise von 60°C bis 120°C, insbesondere von 60°C bis 100°C, erwärmt und auf den unteren Teil der in Schritt a) verwendeten ersten Destillationskolonne gefahren. Zweckmäßigerweise wird zur Erwärmung das aus der Waschkolonne abgepumpte Sumpfprodukt durch einen Wärmtauscher geleitet.

Vorzugsweise wird der erwärmte Sumpfablauf aus Schritt I) auf den 2. bis 15., insbesondere auf den 5. bis 10. Boden, gerechnet vom Kolonnenboden, der ersten Destillationskolonne gegeben.

Durch die Rückführung des erwärmten Sumpfproduktes aus der Waschkolonne aus Schritt e) auf den unteren Teil der in Schritt a) verwendeten ersten Destillationskolonne wird die Temperatur des Sumpfablaufs der Waschkolonne, dessen Temperatur ohne diese Maßnahme im allgemeinen bei 30 bis 50°C liegt, deutlich erhöht. Dabei wird zunächst in einem ersten Schritt der Sumpfablauf, beispielsweise in einem Wärmetauscher, auf eine Temperatur von wenigstens 30°C, vorzugsweise von 60°C bis 120°C und insbesondere von 60°C bis 100°C, erwärmt. Bei der Aufgabe des so erwärmten Sumpfablaufs des Kreisgaswäschers auf den unteren Teil der ersten Destillationskolonne wird dieser Stoffstrom nochmals erwärmt, im allgemeinen auf eine Temperatur von 80°C bis 150°C, was auch der Temperatur des Sumpfablaufs der ersten Destillationskolonne entspricht. Durch dieses Aufheizen des Sumpfablaufs der Waschkolonne verringert sich die Löslichkeit der gasförmigen Komponenten in dem essigsauren, rohen Vinylacetat. Die gasförmigen Komponenten, insbesondere Ethylen und Kohlendioxid, werden verstärkt über den Kopf der ersten Destillationskolonne ausgetrieben und an einer sehr frühen Stelle des Aufarbeitungsprozesses wieder in den Gaskreislauf zurückgeführt. Bei der Entspannung des Rohproduktes fällt daher weniger Gas an. Die Entspannung erfolgt dabei in einem Sammelbehälter, auch Rohvinylacetatsammelbehälter genannt, von einer Druckstufe von 0,5 bis 2,0 MPa auf eine Druckstufe von 0,02 bis 0,2 MPa, vorzugsweise auf 0,1 bis 0,15 MPa. Das bei der Entspannung anfallende Gas bezeichnet man auch als Rückgas und es enthält überwiegend Ethylen und daneben Kohlendioxid und weitere Inerte wie Stickstoff und Argon sowie auch organische Bestandteile wie Essigsäure und geringe Mengen an Vinylacetat und Ethylacetat. Bei der Rückführung des Rückgases in den Prozess ist daher ein geringerer Energieaufwand erforderlich, um das Rückgas wieder auf die Druckstufe des Reaktors zu komprimieren. Durch die erfindungsgemäße Rückführung des Sumpfablaufs aus der Waschkolonne aus Schritt e) wird daher der Rückgasverdichter entlastet, was eine bedeutende Energieeinsparung mit sich bringt.

Weiterhin wird durch die Aufgabe der essigsauren Lösung aus der Waschkolonne auf den unteren Teil der ersten Destillationskolonne, vorzugsweise auf den 2. bis 15. Boden, insbesondere auf den 5. bis 10. Boden, gerechnet vom Kolonnenboden, eine Waschwirkung erzielt. Ethylacetat wird in den Sumpf der ersten Destillationskolonne gewaschen und über den Sumpf ausgeschleust.

Vinylacetat befindet sich in dem Sumpfprodukt der ersten Destillationskolonne, in der in Schritt e) gebildeten essigsauren Waschlösung, die in Schritt l) auf den unteren Teil der ersten Destillationskolonne zurükgeführt wird sowie in dem nicht als Rücklauf in Schritt d) benutzten Anteil der in Schritt b) gebildeten organischen Phase. Der Vinylacetatgehalt in diesen drei Stoffströmen hängt von der Anlagenfahrweise ab und ist für die Durchführung des erfindungsgemäßen Verfahrens nicht kritisch.

Das Kopfprodukt der ersten Destillationskolonne enthält nur noch sehr geringen Mengen an Ethylacetat und der in Schritt d) zurückgeführte Rücklauf sowie der nicht als Rücklauf verwendete Teil der organischen Phase ist ethylacetatarm und kann ohne weitere Maßnahmen, die eine Ethylacetatabtrennung erfordern, weiterverarbeitet werden. Dazu wird nach Schritt m) die abgezogene organische Phase entspannt und die dabei anfallende Flüssigkeit mit der in Schritt i) anfallenden organischen Phase vereinigt, die aus dem Kopfprodukt der zweiten Destillationskolonne, auch Azeotropkolonne genannt, erhalten wird. Die zusammengeführten organischen Phasen werden teilweise als Rücklauf auf den Kopf der Azeotropkolonne zurückgeführt. Der Rest wird auf die dritte Destillationskolonne, auch als Entwässerungskolonne bezeichnet, gegeben (Schritt n).

Das bei der Entspannung in Schritt m) anfallende Gas bezeichnet man auch als Rückgas und weist etwa die gleiche Zusammensetzung auf, wie das in Schritt f) anfallende Rückgas. Beide Rückgasströme werden vereinigt, dann in einem Rückgasverdichter aufkomprimiert und anschließend in den Prozess zurückgeführt. Zweckmäßigerweise wird das vereinigte Rückgas mit dem bei der Essigsäurewäsche in Schritt e) anfallenden Restgas, das man auch als Kreisgas bezeichnet, vereinigt. Die vereinigten Gasströme werden komprimiert und nach Ausschleusung eines Inerte enthaltenden Anteils wieder in den Vinylacetatreaktor zurückgeführt.

In der Gaswäsche von Schritt e) setzt man zumindest teilweise das Sumpfprodukt der zweiten Destillationskolonne (Schritt g)) ein. Das Sumpfprodukt besteht hauptsächlich aus Essigsäure und enthält höchstens 10 Gew.-% Wasser. Ein nicht in Schritt e) benötigter Anteil des Sumpfprodukts wird vorzugsweise als Rückessigsäure zum Reaktor zurückgeführt, nachdem ein kleiner Teil zur Entfernung von Hochsiedern und Polymeren ausgeschleust wurde.

In Schritt n) führt man vorzugsweise nur soviel von der aus Schritt i) und m) zusammengeführten organischen Phase als Rücklauf zurück, dass der Kopfdampf der zweiten Destillationskolonne möglichst wenig Ethylacetat enthält. Denjenigen Teil der organischen Phase, der nicht für diesen Zweck benötigt wird, leitet man in die dritte Destillationskolonne, auch Entwässerungskolonne genannt, ein.

In Schritt o) wird das kondensiert Kopfprodukt der dritten Destillationskolonne nicht ganz als Rücklauf benutzt, sondern es wird ein zur Leichtsieder- und Wasserabtrennung ausreichender Teil abgezogen.

In Schritt p) wird der Sumpfablauf der dritten Destillationskolonne, der nahezu aus trockenem Vinylacetat besteht, auf eine vierte Destillationskolonne, der sogenannten Reinvinylacetatkolonne, an der reines Vinylacetat als Kopfprodukt abgezogen wird, gegeben (Schritt q).

Die in dem beanspruchten Aufarbeitungsverfahren von Vinylacetat benutzte erste, zweite, dritte und vierte Destillationskolonne werden bei solchen Temperatur-, Druck- und Rücklaufverhältnissen betrieben, die sich von der Anlagenauslastung her ergeben.

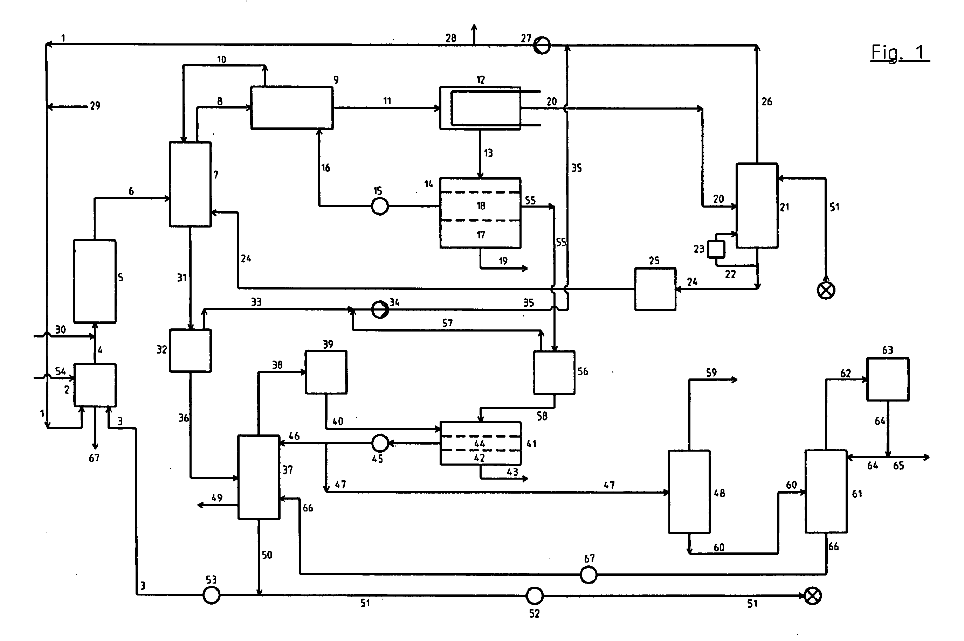

Das erfindungsgemäße Verfahren wird durch Figur 1 erläutert. An sich bekannte Maßnahmen, wie Stabilisatorzusatz, werden nicht dargestellt.

Das aus Ethylen, Sauerstoff und CO₂ sowie Inerten und geringen organischen Anteilen, wie Essigsäure, bestehende zurückgeführte Gasgemisch, auch als Kreisgas bezeichnet, wird über die Leitung (1) in einen als Bodenkolonne ausgebildeten Essigsäureverdampfer (2) geleitet, in dem der Gasstrom mit Essigsäure beladen wird, die über Leitung (3) zugeführt wird. Das den Essigsäureverdampfer (2) verlassende Gasgemisch wird über eine dampfbeheizte Leitung (4) dem Vinylacetatreaktor (5) zugeführt.

Das den Vinylacetatreaktor (5) verlassende Gasgemisch, das im wesentlichen aus Ethylen, Essigsäure, Vinylacetat, Wasser, Kohlendioxid, Sauerstoff sowie inerten Gasen, wie zum Beispiel Stickstoff und Argon besteht, wird über die Leitung (6) in die erste Destillationskolonne, die Vorentwässerungskolonne (7), eingeleitet. Die Vorentwässerungskolonne (7) wird in an sich bekannter Weise ausgelegt.

Das aus der Vorentwässerungskolonne (7) über Kopf austretende Gasgemisch gelangt über Leitung (8) in einen Wärmetauscher (9), wo es in Gegenstromwärmeaustausch mit dem Rücklauf gebracht wird, der über Leitung (16) eintritt und über Leitung (10) in die Vorentwässerungskolonne (7) zurückgeführt wird. Das Gasgemisch gelangt aus dem Wärmetauscher (9) über Leitung (11) in einen wassergekühlten Kondensator (12), in dem es auf etwa 35°C abgekühlt wird. Die dabei verflüssigten Anteile gelangen über Leitung (13) in Behälter (14), wo sie gesammelt werden. Der ein bestimmtes Niveau im Sammelbehälter (14) überschreitende Anteil an Flüssigkeit wird mittels Pumpe (15) über Leitung (16), Wärmetauscher (9) und Leitung (10) in die Vorentwässerungskolonne (7) zurückgepumpt. Nach einiger Zeit trennt sich das im Sammelbehälter (14) anfallende Kondensat in zwei Phasen (17) und (18); von nun an wird die wässrige Phase (17) über Leitung (19) ausgeschleust und nur die organische Phase (18) wird ganz oder teilweise über Leitung (16), Wärmetauscher (9) und Leitung (10) als Rücklauf auf den Kopf der Vorentwässerungskolonne (7) zurückgepumpt.

Das den Kondensator (12) über Leitung (20) verlassende Gasgemisch wird mit der über Leitung (51) herangeführten Essigsäure in der Waschkolonne (21) (Kreisgaswäscher) gewaschen und von nicht kondensierten Vinylacetatanteilen befreit. Der Sumpfablauf aus dem Kreisgaswäscher (21) wird getrennt, wobei ein Teilstrom über die Leitung (22) im Umpump geführt wird und unter Kühlung mittels des Wärmetauschers (23) auf den unteren Teil des Kreisgaswäschers (21) zurückgeführt wird, während der andere Teil des Sumpfablaufs über die Leitung (24) durch einen Wärmetauscher (25) geleitet wird, in dem der Sumpfabzug auf eine Temperatur von wenigstens 30°C, vorzugsweise von 60 bis 120°C und insbesondere von 60 bis 100°C, erwärmt wird. Der so erwärmte Sumpfabzug wird anschließend auf den unteren Teil der Vorentwässerungskolonne (7) zurückgepumpt, vorzugsweise auf den 2. bis 15., insbesondere auf den 5. bis 10. Boden, gerechnet vom Kolonnenboden.

Das die Waschkolonne (21) über Leitung (26) verlassende Restgas oder Kreisgas (Ethylen, nicht umgesetzter Sauerstoff und als Nebenprodukt gebildetes CO₂), wird mit dem über Leitung (35) zugeführten Rückgas, das überwiegend Ethylen und daneben CO₂, Inerte wie Stickstoff und Argon, sowie Essigsäure und geringe Mengen an Vinylacetat und Ethylacetat enthält, vereinigt, mittels des Kreisgaskompressor (27) komprimiert und über Leitung (1) und den Essigsäureverdampfer (2) zum Reaktor (5) zurückgeführt. Ein Teil des Kreisgases wird zur Ausschleusung von inerten Bestandteilen über Leitung (28) als Abgas entfernt. Über Leitung (29) wird frisches Ethylen und über Leitung (30) frischer Sauerstoff zugeführt.

Die am Sumpf der Vorentwässerungskolonne (7) anfallende Flüssigkeit, die hauptsächlich aus Vinylacetat, Essigsäure und Wasser besteht und fast das gesamte Ethylacetat enthält, wird über Leitung (31) einem Behälter (32), auch als Rohvinylacetatsammelbehälter bezeichnet, zugeführt und entspannt, vorzugsweise auf einen Druck von 0,02 bis 0,2 MPa, insbesondere auf einen Druck von 0,1 bis 0,15 MPa. Das sich dabei gebildete Rückgas, das überwiegend Ethylen und daneben CO₂, Inerte wie Stickstoff und Argon, sowie organische Bestandteile, wie Essigsäure enthält, wird über die Leitung (33) abgeführt, mit dem aus Leitung (57) herangeführten Rückgas, das etwa die gleiche Zusammensetzung aufweist, vereinigt, und nach Komprimierung im Rückgasverdichter (34) über die Leitung (35) mit dem über Leitung (26) herangeführten Kreisgas aus dem Kreisgaswäscher (21) vereinigt. Die in dem Rohvinylacetatsammelbehälter (32) nach Entspannung anfallende organische Phase wird über die Leitung (36) abgezogen und in die zweite Destillationskolonne (37), auch Azeotropkolonne genannt, eingeleitet.

Der Kopfdampf der zweiten Destillationskononne (37) wird über Leitung (38) in Kühler (39) geleitet und dort kondensiert. Das über Leitung (40) in den Phasentrenner (41) gelangende Kondensat trennt sich in eine wässrige Phase (42), die über Leitung (43) abgezogen wird und eine organische Phase (44), die mit der aus Leitung (58) herangeführten organischen Phase zusammengeführt wird. Die im Phasentrenner (41) vereinigte organische Phase wird mittels Pumpe (45) abgeführt. Ein Teil der abgeführten organischen Phase wird über die Leitung (46) auf den Kopf der Aezotropkolonne (37) gegeben und dient dort als Rücklauf. Der nicht als Rücklauf verwendete Teil wird über die Leitung (47) abgeführt und auf eine dritte Destillationskolonne (48), die Entwässerungskolonne, gegeben. Das über Leitung (36) in Kolonne (37) gelangende Ethylacetat wird aus einer Anreicherungszone oberhalb des Sumpfes der Kolonne (37) über Leitung (49) abgezogen. Das Sumpfprodukt der Kolonne (37) enthält nahezu vollständig die in der Vinylacetataufarbeitungsstufe anfallende Essigsäure, höchstens 10 Gew.-% Wasser sowie geringe Mengen Hochsieder bzw. Polymere und nur noch Spuren von Vinylacetat und Ethylacetat.

Die wässrige Essigsäure wird aus dem Sumpf der Kolonne (37) über die Leitung (50) abgezogen und aufgeteilt. Je nach Auslegung der Waschkolonne (21) und der Temperatur des zu waschenden Gases werden unterschiedliche Mengen an Essigsäure als Waschflüssigkeit benötigt. Der für die Essigsäurewäsche in Schritt e) benötigte Anteil wird über Leitung (51) und Pumpe (52) der Waschkolonne (21) zugeführt. Der Rest wird über Pumpe (53) und Leitung (3) dem Essigsäureverdampfer (2) zugeführt. Frische Essigsäure wird über Leitung (54) entsprechend der bei der Reaktion verbrauchten Essigsäuremenge auf den Kopf des Essigsäureverdampfers (2) gegeben und dient gleichzeitig als Waschlösung für die über Leitung (3) herangeführte, wiedergewonnenen Essigsäure, die man auch als Rückessigsäure bezeichnet.

Der Rest der organischen Phase (18) aus Sammelbehälter (14) wird über die Leitung (55), falls nicht die gesamte organische Phase (18) als Rücklauf in der Vorentwässerungskolonne (7) verwendet wird, dem Entspannungsbehälter (56) zugeführt. Das bei der Entspannung auf einen Druck von 0,02 bis 0,2 MPa, vorzugsweise auf 0,1 bis 0,15 MPa gebildete Rückgas wird über die Leitung (57) abgeführt, mit dem über Leitung (33) herangeführten Rückgas vereinigt und nach Verdichtung mittels des Rückgasverdichters (34) über die Leitung (35) in den Prozess zurückgeführt.

Die im Behälter (56) anfallende Flüssigkeit wird über Leitung (58) auf den Phasentrenner (41) gegeben, von wo aus die zusammengeführten organischen Phasen zum Teil als Rücklauf über die Leitung (46) auf die Azeotropkolonne (37) und zum Teil als Zufluß über die Leitung (47) auf die dritte Destillationskolonne (48), auch Entwässerungskolonne genannt, gegeben werden. Der Zulauf zur Entwässerungskolonne ist nahezu essigsäurefrei.

Die im Kopfdampf der Kolonne (48) enthaltenen Leichtsieder und letzte Wasserreste werden über die Leitung (59) abgeführt und aus dem Aufarbeitungsprozess ausgeschleust.

Das nahezu wasserfreie Vinylacetat, das am Sumpf der Kolonne (48) anfällt, wird über Leitung (60) zur vierten Destillationskolonne (61), auch Reinvinylacetatkolonne genannt, geführt. Der Kopfdampf dieser Kolonne geht über Leitung (62) zum Kondensator (63). Das anfallende Kondensat ist Ethylacetat freies reines Vinylacetat. Über Leitung (64) wird ein sehr kleiner Teil dieses Vinylacetats als Rücklauf in die Kolonne (61) zurückgeleitet. Über Leitung (65) wird reines Vinylacetat abgezogen. Das Sumpfprodukt der Kolonne (61), das geringe Mengen an Ethylacetat, Polymere und Hochsieder enthält, wird über Leitung (66) und Pumpe (67) zurückgeführt zur Kolonne (37). Aus dem Essigsäureverdampfer (2), in dem letztlich alle Hochsieder und Polymere zurückgeführt werden, wird über Leitung (67) ein Teilstrom zur Polymerenausschleusung abgezogen.

Die für das erfindungsgemäße Aufarbeitungsverfahren wesentliche Maßnahme besteht in der Rückführung des erwärmten, essigsauren Sumpfablaufs aus der Waschkolonne (21) in den unteren Teil der Vorentwässerungskolonne (7), wobei sich der schon erwärmte, essigsaure Sumpfablauf aus der Waschkolonne nochmals erwärmt. Überraschenderweise ist diese Maßnahme mit vielfältigen Vorteilen verbunden.

Durch diese Maßnahme erniedrigt sich die Löslichkeit der in dem Sumpfablauf des Kreisgaswäschers enthaltenen gasförmigen Komponenten, insbesondere von Ethylen und Kohlendioxid, die über den Kopf der ersten Destillationskolonne ausgetrieben werden und an einer frühen Prozessstufe wieder in das Kreisgas zurückgeschleust werden.

Dadurch fällt bei der Entspannung weniger Rückgas an, das unter geringerem Energieaufwand in dem Rückgasverdichter (34) aufkomprimiert und in den Prozess zurückgeschleust wird. Der Rückgasverdichter wird somit entlastet.

Durch die Aufgabe des erwärmten, essigsauren Sumpfablaufs aus der Waschkolonne (21) auf den unteren Teil der Vorentwässerungskolonne (7) wird eine Waschwirkung erzielt und nahezu das gesamte Ethylacetat wird in den Sumpf der Vorentwässerungskolonne (7) gewaschen und über den Sumpfablauf ausgeschleust. Nur ein sehr geringer Anteil an Ethylacetat wird in die organische Phase (18), die sich im Sammelbehälter (14) ansammelt, mitgeschleppt. Der über Leitung (55) abgezogene Stoffstrom enthält daher kaum noch Ethylacetat. Sein Anteil kann daher im Vergleich zu der bekannten Arbeitsweise verstärkt gefahren werden, wodurch die Belastung der Azeotropkolonne (37) gemindert wird, was ebenfalls zu weiteren Energieeinsparungen führt. So kann die Azeotropkolonne mit einem deutlich niedrigeren Rücklaufverhältnis betrieben werden im Vergleich zu der bekannten Arbeitsweise.

Ebenfalls wird eine im Vergleich zu der bekannten Arbeitsweise größere Wassermenge über den Kopf der Vorentwässerungskolonne (7) abgeführt, wodurch sich der Wasseranfall in dem Behälter (14) erhöhen lässt. Daher kann die Wasserentfernung über die Vorentwässrungskolonne (7) effektiver betrieben werden. Wasser wird somit an einer frühen Prozessstufe verstärkt entfernt und spätere Prozessstufen zur Wasserentfernung werden entlastet.

Ferner wird die gesamte Essigsäure mit dem Sumpf der Azeotropkolonne (37) abgeführt, so dass der Zulauf zur Entwässerungskolonne (48) und damit auch zur Reinvinylacetatkolonne (61) essigsäurefrei ist. Daher lassen sich in diesen Anlagenteilen durch Essigsäure verursachte Korrosionserscheinungen vermeiden und es können weniger korrosionsbeständige Materialien verbaut werden. Durch die Vermeidung eines Essigsäuregehalts in dem Zulauf zur Reinvinylacetatkolonne (61) verringert sich auch der Destillationsaufwand zur Gewinnung des reinen Vinylacetats, da die Abtrennung von Restspuren von Essigsäure aus Vinylacetat sehr aufwendig ist. Die Destillation zur Gewinnung des reinen Vinylacetats kann daher unter geringerem Energieeinsatz und geringerem Rücklaufverhältnis betrieben werden, was im allgemeinen eine erhebliche Dampfeinsparung bedeutet.

## Patentansprüche

1. Verfahren zur Abtrennung von Vinylacetat aus dem Gasgemisch, das bei der Umsetzung von Ethylen mit Essigsäure und Sauerstoff an Palladium oder Palladiumverbindungen enthaltenden Katalysatoren in der Gasphase gebildet wird, wobei man
a) das aus der Reaktionszone austretende Gasgemisch in eine erste Destillationskolonne einleitet,
b) das am Kopf der ersten Destillationskolonne austretende Gasgemisch auf -20 bis +50°C abkühlt, wobei sich das anfallende Kondensat in eine Wasserphase und in eine organische Phase trennt,
c) die in Schritt b) gebildete Wasserphase abzieht,
d) die in Schritt b) gebildete organische Phase ganz oder teilweise als Rücklauf auf den Kopf der in Schritt a) benutzten ersten Destillationskolonne zurückführt und einen nicht als Rücklauf verwendeten Teil der organischen Phase abzieht,
e) das in Schritt b) nicht kondensierte, Vinylacetat haltige Gas in einer Waschkolonne mit mindestens 90%iger wässriger Essigsäure wäscht und dabei am Sumpf eine Vinylacetat haltige, essigsaure Lösung gewinnt,
f) das Vinylacetat, Ethylacetat, Essigsäure und Wasser enthaltende Sumpfprodukt von Schritt a) einem Sammelbehälter zuführt und die unter Druck stehende Flüssigkeit entspannt, wobei sich ein Gas bildet,
g) die bei der Entspannung in Schritt f) anfallende Flüssigkeit einer zweiten Destillationskolonne zuführt und aus einer Anreicherungszone oberhalb von deren Sumpf einen Ethylacetat haltigen Seitenstrom abzieht,
h) das Essigsäure und Wasser enthaltende Sumpfprodukt von Schritt g) ganz oder teilweise zur Gaswäsche in Schritt e) benutzt,
i) den Kopfdampf von Schritt g) abkühlt, wobei sich das anfallende Kondensat in eine wässrige und eine organische Phase trennt,
j) die in Schritt i) gebildete wässrige Phase abzieht,
k) einen Teil der in Schritt i) gebildeten organischen Phase als Rücklauf auf den Kopf der in Schritt g) benutzten zweiten Destillationskolonne zurückführt und den restlichen Teil abzieht, **dadurch gekennzeichnet, dass** man
l) einen Teil des Sumpfproduktes der in Schritt e) benutzten Waschkolonne unter Umpump zunächst kühlt und in den Sumpf der in Schritt e) benutzten Waschkolonne zurückführt, den restlichen Teil abzieht und den abgezogenen Teil auf eine Temperatur von wenigstens 30°C erwärmt und auf den unteren Teil der in Schritt a) benutzten ersten Destillationskolonne gibt,
m) die in Schritt d) abgezogene restliche organische Phase entspannt, das bei der Entspannung gebildete Gas mit dem in Schritt f) gebildeten Gas vereinigt und das gemeinsame Gas wieder in den Prozess zurückführt,
n) die in Schritt m) anfallende organische Phase mit der in Schritt i) anfallenden organischen Phase vereinigt und den in Schritt k) restlichen abgezogenen, nicht als Rücklauf verwendeten Teil der organischen Phase in eine dritte Destillationskolonne einleitet,
o) das Kopfprodukt der dritten Destillationskolonne aus Schritt n) abkühlt und die dabei anfallenden Leichtsieder und das dabei anfallende Wasser abtrennt,
p) das Sumpfprodukt der dritten Destillationskolonne aus Schritt n) in eine vierte Destillationskolonne einleitet,
q) am Kopf der in Schritt p) verwendeten vierten Destillationskolonne reines Vinylacetat abzieht.

2. Verfahren nach Anspruch 1, **dadurch gekennzeichnet, dass** man in Schritt l) das aus der Waschkolonne abgezogene Sumpfprodukt auf eine Temperatur von 60°C bis 120°C, insbesondere von 60°C bis 100°C, erwärmt.

3. Verfahren nach Anspruch 1 oder 2, **dadurch gekennzeichnet, dass** man das in Schritt l) abgezogene Sumpfprodukt auf den 2. bis 15., vorzugsweise auf den 5. bis 10. Boden, gerechnet vom Kolonnenboden, der in Schritt a) benutzten ersten Destillationskolonne, gibt.

4. Verfahren nach einem oder mehreren der Ansprüche 1 bis 3, **dadurch gekennzeichnet, dass** die Rückführung des in Schritt l) abgezogenen Sumpfproduktes in die in Schritt a) benutzte erste Destillationskolonne in der Weise erfolgt, dass der Sumpfablauf der ersten Destillationskolonne eine Temperatur von 80°C bis 150°C aufweist.

5. Verfahren nach einem oder mehreren der Ansprüche 1 bis 4, **dadurch gekennzeichnet, dass** man in Schritt f) das Sumpfprodukt von Schritt a) in dem Sammelbehälter auf einen Druck von 0,02 bis 0,2 MPa, vorzugsweise auf 0,1 bis 0,15 MPa, entspannt.

6. Verfahren nach einem oder mehreren der Ansprüche 1 bis 5, **dadurch gekennzeichnet, dass** man in Schrit m) eine in Schritt d) abgezogene restliche organische Phase einem Entspannungsbehälter zuführt und auf einen Druck von 0,02 bis 0,2 MPa, vorzugsweise auf 0,1 bis 0,15 MPa entspannt.

7. Verfahren nach einem oder mehreren der Ansprüche 1 bis 6, **dadurch gekennzeichnet, dass** man die Abkühlungstemperatur in Schritt b) und den als Rücklauf in Schritt d) benutzten Anteil der in Schritt b) gebildeten organischen Phase derart wählt, dass möglichst das gesamte Ethylacetat im Sumpfprodukt der ersten Destillationskolonne von Schritt a) enthalten ist.

8. Verfahren nach einem oder mehreren der Ansprüche 1 bis 7, **dadurch gekennzeichnet, dass** man den in Schritt d) nicht als Rücklauf verwendeten Teil der organischen Phase erhöht, wobei die in Schritt n) in einem Phasentrenner vereinigten organischen Phasen möglichst wenig Ethylacetat enthalten.

9. Verfahren nach einem oder mehreren der Ansprüche 1 bis 8, **dadurch gekennzeichnet, dass** in Schritt n) nur soviel von der vereinigten organischen Phase als Rücklauf zurückgeführt wird, dass der Kopfdampf der zweiten Destillationskolonne möglichst wenig Essigsäure und Ethylacetat enthält.

10. Verfahren nach einem oder mehreren der Ansprüche 1 bis 9, **dadurch gekennzeichnet, dass** man in Schritt o) soviel von dem abgekühlten Kopfprodukt als Rücklauf auf die dritte Destillationskolonne aufgibt, um einen ausreichenden Teil Leichtsieder und Wasser abzutrennen.

11. Verfahren nach einem oder mehreren der Ansprüche 1 bis 10, **dadurch gekennzeichnet, dass** man in Schritt a) das aus der Reaktionszone austretende Gasgemisch zunächst im Gegenstromwärmeaustauscher mit dem kälteren Kreisgas auf 115°C bis 150°C abkühlt und erst dann in die erste Destillationskolonne einleitet.

## Claims

1. A process for separating off vinyl acetate from a gas mixture formed in the reaction of ethylene with acetic acid and oxygen in the gas phase over catalysts comprising palladium or palladium compounds, comprising the following steps:
a) introducing the gas mixture leaving the reaction zone into a first distillation column;
b) cooling the gas mixture leaving the top of the first distillation column to from -20 to +50°C, with the condensate obtained separating into a water phase and an organic phase;
c) taking off the water phase formed in step b);
d) recirculating all or part of the organic phase formed in step b) as runback to the top of the first distillation column utilized in step a) and taking off part of the organic phase which is not used as runback;
e) scrubbing the gas comprising vinyl acetate which is not condensed in step b) in a scrubbing column by means of at least 90% strength aqueous acetic acid and obtaining an acetic-acid solution comprising vinyl acetate at the bottom;
f) feeding the bottom product comprising vinyl acetate, ethyl acetate, acetic acid and water from step a) into a collection vessel and depressurizing the pressurized liquid so as to form a gas;
g) feeding the liquid obtained in the depressurization in step f) into a second distillation column and taking off a side stream comprising ethyl acetate from an enrichment zone above its bottom;
h) utilizing all or part of the bottom product comprising acetic acid and water from step g) for the gas scrub in step e);
i) cooling the overhead vapor from step g), with the condensate obtained separating into an aqueous phase and an organic phase;
j) taking off the aqueous phase formed in step i);
k) recirculating part of the organic phase formed in step i) as runback to the top of the second distillation column utilized in step g) and taking off the remainder; **characterized in that**
l) part of the bottom product from the scrubbing column utilized in step e) is firstly cooled with pumped circulation and recirculated to the bottom of the scrubbing column utilized in step e), the remainder is taken off and the part taken off is heated to a temperature of at least 30 °C and fed into the lower part of the first distillation column utilized in step a);
m) the remaining organic phase taken off in step d) is depressurized, the gas formed in the depressurization is combined with the gas formed in step f) and the combined gas is returned to the process;
n) the organic phase obtained in step m) is combined with the organic phase obtained in step i) and the remaining part of the organic phase taken off in step k) which has not been used as runback is introduced into a third distillation column;
o) the overhead product from the third distillation column in step n) is cooled and the low boilers obtained and the water obtained are separated off;
p) the bottom product from the third distillation column in step n) is introduced into a fourth distillation column;
q) pure vinyl acetate is taken off at the top of the fourth distillation column used in step p).

2. The process of claim 1, **characterized in that** the bottom product taken off from the scrubbing column is heated to a temperature of from 60°C to 120°C, preferably from 60°C to 100°C, in step l).

3. The process of claim 1 or 2, **characterized in that** the bottom product taken off in step l) is fed into the first distillation column utilized in step a) at the 2nd to 15th plate, preferably at the 5th to 10th plate, calculated from the bottom of the column.

4. The process of one or more of claims 1 to 3, **characterized in that** the recirculation of the bottom product taken off in step l) to the first distillation column utilized in step a) is carried out in such a way that the outflow from the bottom of the first distillation column has a temperature of from 80°C to 150°C.

5. The process of one or more of claims 1 to 4, **characterized in that** the bottom product from step a) is depressurized to a pressure of from 0.02 to 0.2 MPa, preferably from 0.1 to 0.15 MPa, in the collection vessel in step f).

6. The process of one or more of claims 1 to 5, **characterized in that** a residual organic phase taken off in step d) is fed to a depressurization vessel and depressurized to a pressure of from 0.02 to 0.2 MPa, preferably from 0.1 to 0.15 MPa, in step m).

7. The process of one or more of claims 1 to 6, **characterized in that** the cooling temperature in step b) and the proportion of the organic phase formed in step b) which is utilized as runback in step d) are selected so that virtually all the ethyl acetate is present in the bottom product from the first distillation column of step a).

8. The process of one or more of claims 1 to 7, **characterized in that** the part of the organic phase which is not used as runback in step d) is increased, with the organic phases combined in a phase separator in step n) containing as little ethyl acetate as possible.

9. The process of one or more of claims 1 to 8, **characterized in that** in step n) the amount of the combined organic phase which is recirculated as runback is only the amount required for the overhead vapor from the second distillation column to contain as little acetic acid and ethyl acetate as possible.

10. The process of one or more of claims 1 to 9, **characterized in that** the amount of the cooled overhead product returned as runback to the third distillation column in step o) is the amount necessary for a sufficient part of the low boilers and water to be separated off.

11. The process of one or more of claims 1 to 10, **characterized in that** the gas mixture leaving the reaction zone is firstly cooled to from 115°C to 150°C by means of the colder circulating gas in the countercurrent heat exchanger and only then introduced into the first distillation column in step a).

## Revendications

1. Procédé pour séparer l'acétate de vinyle du mélange de gaz formé pendant la réaction d'éthylène avec l'acide acétique et oxygène sur des catalyseurs contenant du palladium ou des composés de palladium dans la phase gazeuse, dans lequel
a) le mélange de gaz quittant la zone réactionnelle est introduit dans une première colonne de distillation;
b) le mélange de gaz quittant la tête de la première colonne de distillation est refroidi à une température de -20 à +50°C, le condensat obtenu se séparant en une phase aqueuse et une phase organique;
c) la phase aqueuse formée dans l'étape b) est entraînée;
d) la phase organique formée dans l'étape b) est ramenée partiellement ou entièrement en tant de produit de retour à la tête de la première colonne de distillation utilisée dans l'étape a), et une partie de la phase organique qui n'est pas utilisée en tant de produit de retour est entraînée;
e) le gaz contenant de l'acétate de vinyle qui n'est pas condensé dans l'étape b) est lavé dans une colonne de lavage avec de l'acide acétique aqueux à au moins 90%, récupérant une solution contenant de l'acétate de vinyle et de l'acide acétique au fond de la colonne;
f) le produit de fond contenant de l'acétate de vinyle, de l'acétate d'éthyle, de l'acide acétique et de l'eau obtenu dans l'étape a) est dirigé à un récipient collecteur, et le liquide sous pression est détendu pour former un gaz;
g) le liquide obtenu lors de la détente dans l'étape f) est dirigé à une seconde colonne de distillation, et un courant latéral contenant de l'acétate d'éthyle est prélevé d'une zone d'enrichissement au-dessus du fond de la colonne;
h) le produit de fond contenant de l'acide acétique et de l'eau et obtenu dans l'étape g) est partiellement ou entièrement utilisé pour laver le gaz dans l'étape e);
i) la vapeur de tête obtenu dans l'étape g) est refroidie, le condensat obtenu séparant en une phase aqueuse et une phase organique;
j) la phase aqueuse formée dans l'étape i) est entraînée;
k) une partie de la phase organique formée dans l'étape i) est ramenée en tant de produit de retour à la tête de la seconde colonne de distillation utilisée dans l'étape g), et la partie restante est entraînée, **caractérisé en ce que** l'on
l) refroidit une partie du produit de fond de la colonne de lavage utilisée dans l'étape e) au préalable avec circulation forcée et la ramène dans le fond de la colonne de lavage utilisée dans l'étape e), entraîne la partie restante et réchauffe la partie entraînée à une température d'au moins 30 °C et l'alimente à la partie inférieure de la première colonne de distillation utilisée dans l'étape a);
m) détend la phase organique restante entraînée dans l'étape d), combine le gaz formé lors de la détente avec le gaz formé dans l'étape f), et recycle le gaz combiné dans le processus;
n) combine la phase organique obtenue dans l'étape m) avec la phase organique obtenue dans l'étape i), et introduit la partie de la phase organique entraînée dans l'étape k), restante, qui n'a pas été utilisée en tant de produit de retour, dans une troisième colonne de distillation;
o) refroidit le produit de tête de la troisième colonne de distillation obtenu dans l'étape n), et sépare les fractions légères obtenues par ce moyen et l'eau obtenue par ce moyen;
p) introduit le produit de fond de la troisième colonne de distillation obtenu dans l'étape n) dans une quatrième colonne de distillation;
q) récupère de l'acétate de vinyle pur de la tête de la quatrième colonne de distillation utilisée dans l'étape p).

2. Procédé selon la revendication 1, **caractérisé en ce que** dans l'étape l), le produit de fond entraîné de la colonne de lavage est réchauffé à une température entre 60 °C et 120 °C, notamment entre 60 °C et 100 °C.

3. Procédé selon la revendication 1 ou 2, **caractérisé en ce que** le produit de fond entraîné dans l'étape l) est alimenté au 2^{ème} à 15^{ème}, préférablement au 5^{ème} à 10^{ème}, plateau, calculé à partir du fond de la colonne, de la première colonne de distillation utilisée dans l'étape a).

4. Procédé selon une ou plusieurs des revendications 1 à 3, **caractérisé en ce que** le recyclage du produit de fond entraîné dans l'étape l) dans la première colonne de distillation utilisée dans l'étape a) est effectué de manière que l'égout de fond de la première colonne de distillation ait une température de 80°C à 150°C.

5. Procédé selon une ou plusieurs des revendications 1 à 4, **caractérisé en ce que,** dans l'étape f), le produit de fond issu de l'étape a) est détendu dans le récipient collecteur à une pression de 0,02 à 0,2 MPa, préférablement de 0,1 à 0,15 MPa.

6. Procédé selon une ou plusieurs des revendications 1 à 5, **caractérisé en ce que,** dans l'étape m), une phase organique restante entraînée dans l'étape d) est alimentée à un récipient de détente et détendue à une pression de 0,02 à 0,2 MPa, préférablement de 0,1 à 0,15 MPa.

7. Procédé selon une ou plusieurs des revendications 1 à 6, **caractérisé en ce que** la température de refroidissement dans l'étape b) et la fraction de la phase organique formée dans l'étape b) qui est utilisée en tant de produit de retour dans l'étape d) sont choisies de manière qu'autant acétate d'éthyle que possible soit contenu dans le produit de fond de la première colonne de distillation de l'étape a).

8. Procédé selon une ou plusieurs des revendications 1 à 7, **caractérisé en ce que** la fraction de la phase organique qui n'est pas utilisée en tant de produit de retour dans l'étape d) est augmentée, les phases organiques combinées dans un séparateur de phases dans l'étape n) contenant aussi peu acétate d'éthyle que possible.

9. Procédé selon une ou plusieurs des revendications 1 à 8, **caractérisé en ce que,** dans l'étape n), la quantité des phases organiques combinées recyclée en tant de produit de retour est limitée de manière que la vapeur de tête de la seconde colonne de distillation contienne aussi peu acide acétique et acétate d'éthyle que possible.

10. Procédé selon une ou plusieurs des revendications 1 à 9, **caractérisé en ce que,** dans l'étape o), autant produit de tête refroidi est alimenté en tant de produit de retour à la troisième colonne de distillation qu'une quantité suffisante de fractions légères et d'eau soit séparée.

11. Procédé selon une ou plusieurs des revendications 1 à 10, **caractérisé en ce que,** dans l'étape a), le mélange de gaz quittant la zone réactionnelle est refroidi au préalable à une température de 115°C à 150°C dans un échangeur de chaleur à contre-courant au moyen du gaz recyclé plus froid, et que ce n'est qu'ensuite que l'on l'introduit dans la première colonne de distillation.
